(19) Europäisches Patentamt
European Patent Office
Office européen des brevets

(11) Publication number: **0 433 683 A2**

(12) # EUROPEAN PATENT APPLICATION

(21) Application number: **90122180.4**

(22) Date of filing: **20.11.90**

(51) Int. Cl.[5]: **A61K 31/55**, //(A61K31/71, 31:55)

The title of the invention has been amended (Guidelines for Examination in the EPO, A-III, 7.3).

(30) Priority: **22.11.89 US 441083**

(43) Date of publication of application: **26.06.91 Bulletin 91/26**

(84) Designated Contracting States: **AT BE CH DE DK ES FR GB GR IT LI LU NL SE**

(71) Applicant: **MARION MERRELL DOW INC.**
**9300 Ward Parkway**
**Kansas City, Missouri 64114(US)**

(72) Inventor: **Ahmed, Nahed K.**
**8812 Rosewood**
**Prairie Village, Kansas 66207(US)**

(74) Representative: **Vossius & Partner**
**Siebertstrasse 4 P.O. Box 86 07 67**
**W-8000 München 86(DE)**

(54) **Use of benzothiazepines as potentiators of anti-cancer drugs.**

(57) Certain benzothiazepines are cancer drug potentiators.

EP 0 433 683 A2

## ANTI-CANCER DRUG POTENTIATORS

This invention is directed to the use of benzothiazepines for cancer treating methods and compositions.

The expression and proliferation of drug-resistant tumor cells, i.e., tumor cells which cytotoxic agents have no appreciable ability to kill at concentrations that are tolerable to normal host tissue, are considered to be a major cause of failure in cancer chemotherapy. Generally, resistance is acquired through multiple mechanisms under the selection pressure of chemotherapy. Multi-drug resistance (MDR) characterizes a complex cell phenotype, the predominant feature of which is resistance to a wide range of cytotoxic agents, many of which are anti-cancer drugs. The in vivo MDR can be mimicked in vitro by developing and selecting resistant cell lines. Although resistance is developed to a single drug, the cells show cross-resistance to a variety of structurally unrelated compounds. The factors underlying the development of resistance are multiple and include: 1) over-expression of a membrane glycoprotein, gp 170; 2) alteration in drug uptake; 3) alteration in drug binding to target sites; 4) increased efflux of drug; 5) alteration in cellular metabolism resulting in activation or inactivation of drug, and 6) alterations in DNA repair mechanisms.

Despite the complexity of MDR and the heterogeneity of tumor cells, sufficient clues suggest the use of some calcium channel blockers (CCBs) as therapeutic possibilities for circumventing drug resistance. See e.g., Helson, Cancer Drug Delivery, 1(4), 353-61 (1984).

Studies have shown that certain phenothiazines, CCBs, and calmodulin inhibitors enhance resistant cells chemosensitivity both in vitro and in vivo. The exact mechanism by which thes agents circumvent resistance is not clear but may be due to increased intra-cellular drug levels or decreased drug efflux. The CCBs were found to be generally more effective than are calmodulin inhibitors, especially with in vivo experiments. Many CCBs, to include verapamil, diltiazem, nicardipine, and nifedipine, have been studied in vivo and in vitro. However, for clinical application, verapamil was the most studied candidate of the CCBs for use with anti-cancer drugs.

Verapamil circumvents drug resistance in a variety of human and murine tumor systems both in vivo and in vitro. Generally, this effect is not mediated with alterations in calcium flux and probably derives from competition for the efflux or binding of drug.

However, the clinical utility of the combination therapy of verapamil with anti-cancer drugs is limited due to verapamil toxicity in man. Oral ingestion of 2 and 5.6 g/kg doses of verapamil by adults has been shown to be accompanied by cardiac toxicity. These concentrations are lower than those generally required to promote drug responsiveness in tumor-bearing mice.

Therefore, the art sought new CCB potentiators.

Diltiazem hydrochloride, a CCB, potentiates the antitumor activity of vinca alkaloids in human and murine tumor-resistant cells. It has been shown to be either less effective or as effective, depending upon tumor type, as verapamil in circumventing drug resistance. However, it was much less toxic than verapamil. A 54-fold increase in vincristine (VCR) cytotoxicity occurred in a VCR-resistant cell line with a nontoxic concentration of diltiazem hydrochloride, demonstrating good circumvention of VCR resistance by it.

The in vivo effect of VCR and diltiazem hydrochloride on VCR-resistant tumor-bearing mice has also been examined. Tsurno et al., Cancer Research, 43, 2905-10 (1983). A 40 to 50 percent increase in life span occurred with a combination of VCR and diltiazem hydrochloride.

Bessho et al., Medical and Pediatric Oncology, 13, 199-202 (1985), reports on a very small clinical trial of five pediatric patients with acute lymphocytic leukemia in Japan using diltiazem in combination with VCR. In four of the five children who took the drug as scheduled, various degrees of tumor cell destruction was reported to have been observed. The authors concluded that diltiazem could partially overcome VCR resistance in a clinical setting, that their findings warranted further clinical trials and that further studies are needed to determine the optimal dosage and administration schedule of diltiazem and optimal timing of anti-cancer drugs.

In spite of these efforts, the art yet lacks and needs CCB anti-cancer drug potentiators. The art lacks and needs CCB anti-cancer drug potentiators that especially provide good or better potentiating effects with adequate or better toxicity profiles than that of the known art.

This invention, in one aspect, provides for the use of a benzothiazepine for preparing a pharmaceutical composition for treating cancer by potentiating an anti-cancer drug effect in a subject.
The composition is administering to the subject in general concurrence amounts of a Benzothiazepine-potentiator and an anti-cancer drug such that the effect of the anti-cancer drug is potentiated. Another aspect provides a pharmaceutical composition useful for treatment of a cancer comprising a Benzothiazepine-potentiator in combination with an anti-cancer drug.

The present invention is useful in cancer treatment.

Notably, the method and composition embodiments of this invention can provide good, if not oftimes excellent, potentiating effects. It can further provide such with a good, if not oftimes excellent, toxicity profile as well.

The drawings form part of the specification hereof.

**Figs. 1, 3, 5, 7, 9, 11, 13, 15, 17 & 19** are graphs of cell density (mean percent of control) on the ordinate vs. daunorubicin concentration on the abscissa, which show the effect of different potentiators on daunorubicin toxicity in K562R/III cells at certain concentrations. The potentiator verapamil **1000** is a comparative and is not of this invention. Daunorubicin 100 is also illustrated.

**Figs. 2, 4, 6, 8, 10, 12, 14, 16, 18 & 20** are graphs of cell density (relative percent of control) on the ordinate vs. daunorubicin concentration on the abscissa, which show the effect of different potentiators on daunorubicin toxicity in K562R/III cells at certain concentrations. The potentiator verapamil **1000** is a comparative and is not of this invention. Daunorubicin **100** is also illustrated.

In **Figs. 1 & 2**, the potentiator of this invention is d-cis-ML1013 fumerate, numerically identified **1013**.

In **Figs. 3 & 4**, the potentiator of this invention is d-cis-ML1014 fumerate, numerically identified 1014.

In **Figs. 5 & 6**, the potentiator of this invention is d-cis-ML1015 fumerate, numerically identified **1015**.

In Figs. 7 & 8, the potentiator of this invention is d-cis-ML1016 hydrochloride, numerically identified **1016**.

In **Figs. 9 & 10**, the potentiator of this invention is dl-cis-ML1021 hydrochloride, numerically identified **1021**.

In Figs. **11 & 12**, the potentiator of this invention is dl-cis-ML1078 hydrochloride, numerically identified **1078**.

In Figs. **13 & 14**, the potentiator of this invention is dl-cis-ML1082 hydrochloride, numerically identified **1082**.

In Figs. **15 & 16**, the potentiator of this invention is dl-trans-ML1103 hydrochloride, numerically identified 1103.

In **Figs. 17 & 18**, the potentiator of this invention is dl-cis-ML1104 hydrochloride, numerically identified as **1104**.

In **Figs. 19 & 20**, the potentiator of this invention is l-cis-TA3090 maleate, numerically identified **3090**.

The terms "potentiating" or "potentiate" or "potentiated" and so forth refer herein to the ability of a compound or composition to circumvent anti-cancer drug resistance of an anti-cancer drug resistant or an MDR cell line. A cell line known in the art, which is useful for determining potentiation of a compound or composition, is the K562/III leukemia cell line, employing resistant versus nonresistant cell types. See, Ahmed & Vasanthakumar, Eur. J. Cancer Clin. Oncol., 23(9), 1329-36 (1987). An IC50 value can be used to quantitate potentiating effect. The IC50 value is defined as the concentration of drug inhibiting the growth of cells to such an extent that their growth is reduced to one-half that observed under drug-free conditions. For substantial potentiating effect, the IC50 value should be eighty percent or less of the value of drug alone, advantageously fifty percent or less, or forty-five percent or less, and desirably forty percent or less.

The term "anti-cancer drug effect" refers herein to a reduction of cell density caused by a drug, without potentiator candidate, in a suitable cell sample. The IC50 value as generally described above can be employed to quantitate the anti-cancer drug effect.

The term "administering" and so forth refers herein to suitably providing an amount of compound or composition in vitro or in vivo to a subject so that a therapeutic effect might result. The administering can be by mere contact of the components with a cell line, or can be by conventional dosage formats as for a mammalian subject. The conventional dosage formats include the ingestion of an oral or sublingual dosage form such as a powder, sample of beads, tablet, capsule, syrup or dragee, the injection of an intravenous solution or colloidal mixture, the application of a transdermal or other external preparation such as a solution, creme, gel or other ointment, and/or the implantation of a rapid or sustained release device.

The term "subject" refers herein to an organism, or organ, organ system or cell line of an organism, which has a cancer cell line living therein or is cancerous. As such, the subject may be treated in vitro or in vivo. The subject is desirably a mammal to include a human patient with in vivo treatment being undertaken.

The term "general concurrence" refers herein to administering of the Benzothiazepine-potentiator and the anti-cancer drug during at least the same general time frame, if not concurrently. Simultaneous administration of the Benzothiazepine-potentiator and the anti-cancer drug can generally be provided by administration of composition embodiments of this invention. However, it may be desirable to administer the Benzothiazepine-potentiator at a different time than the administration of the anti-cancer drug, for example, after the anti-cancer drug is administered. This may be effected by separate administrations of the Benzothiazepine-potentiator and the anti-cancer drug or by delayed release of the Benzothiazepine-potentiator or the anti-cancer drug in relation to the other in composition embodiments of this invention.

The term "Benzothiazepine-potentiator" refers herein to a benzothiazepine compound to include suitable pharmaceutically acceptable salts thereof, excepting for diltiazem, i.e., d-cis-5-[2-(dimethylamino)-ethyl]-2,3-dihydro-3-hydroxy-2-(4-methoxyphenyl)-1,5-benzothiazepin-4(5H)-one acetate (ester), and its pharmaceutically acceptable salts, e.g., its hydrochloride, which potentiates the anti-cancer drug effect of an anti-cancer drug administered in general concurrence therewith. Desirably, other therapeutic effect(s) such as at least one of anti-hypertensive effect, anti-convulsant effect, and anti-depressant effect are substantially low or absent in the practice of this invention with respect to the Benzothiazepine-potentiator in particular.

The Benzothiazepine-potentiator can be selected from a compound represented by the following general formula:

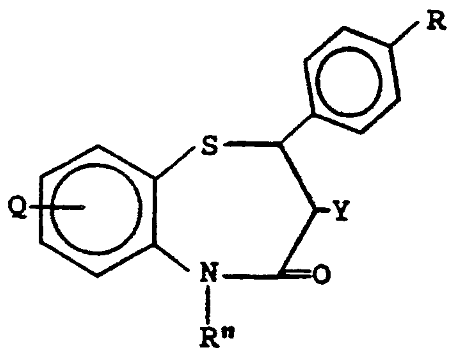

(I)

wherein

Q is hydro (H) or halo to include fluoro (F) and chloro (Cl), especially H or 8-Cl;

R is H, lower alkoxy, lower haloalkyl, cyano (CN), lower alkyl to include methyl (CH3) or halo to include F & Cl, especially H, methoxy (OMe), trifluoromethyl (CF3) or CN;

Y is

$OR'$, wherein $R'$ is H or alkylacyl to include, e.g., lower alkylacyl and adamantylcarboxy, etc., especially H, or lower alkylacyl to include groups such as acetyl, propionyl, butyryl, pivalyl, valeryl, isovaleryl, etc., provided that then there is full saturation between carbons 2 & 3 of the benzothiazepine nucleus and 2,3-dihydro-functionality thereat as well, or

Cl, provided that then there is ethylenic unsaturation between positions 2 & 3 of the benzothiazepine nucleus, and

$R''$ is 2-[di(lower alkyl)amino]ethyl ($R''1$),

3-[di(lower alkyl)amino]propyl ($R''2$), 2-(pyrrolidino)ethyl ($R''3$), 3-(pyrrolidino)propyl ($R''4$), 2-(piperidino)ethyl ($R''5$), 3-(piperidino)propyl ($R''6$), 2-(morpholino)ethyl ($R''7$), 3-(morhpolino)propyl ($R''8$) or (N-pyridinium)alkyl with a suitable counterion being present ($+R''9$-X), especially $R''1$, e.g., with $R''1$ being 2-(dimethylamino)-ethyl ($R''1a$) or with $R''1$ being 2-(diisopropylamino)ethyl ($R''1b$), or $R''3$, or $R''5$, or $+R''9$-X, e.g., with $+R''9$-X being 2-(N-pyridinium)ethyl with a bromide and/or chloride counterion being present ($+R''9a$-X).

Generally, with respect to the 2,3-dihydro compounds, the cis-configuration, i.e., about positions 2 & 3 of the benzothiazepine nucleus, is or can be present, with some exceptions to this, e.g., with the trans-configuration, i.e., about positions 2 & 3 of the benzothiazepine nucleus, being present in the cases of Q being H or 8-Cl, R being methoxy, $R'$ being H, and $R''$ being $R''5$. Also generally, if the compound has a chiral carbon, racemates or separate optical antipodes may be present, with some exceptions to this, e.g., the levorotary optical antipode corresponding to diltiazem being one such exception, a compound employable in the practice of this invention.

Suitable pharmaceutically acceptable salts generally include the hydrochloride, the fumerate, the maleate, the sulfate, the citrate, and so forth.

Preferably, the Benzothiazepine-potentiator is a compound or pharmaceutically acceptable salt selected from cis-TA3090 especially l-cis-TA3090, d-cis-ML1013, d-cis-ML1014, d-cis-ML1015, d-cis-ML1016, cis-ML1021, cis-ML1078, d-cis-ML1082, d-trans-ML1103, and d-cis-ML1104. Also of note, the Benzothiazepine-potentiator may advantageously be l-cis-DTZ or pharmaceutically acceptable salt thereof.

The Benzothiazepine-potentiators can be made by reacting a suitable glycidic acid ester with a suitable aminothiophenol to prepare corresponding aminophenylthiopropionic acid ester, then cyclyzing the latter ester or its corresponding free acid, followed by N-alkylation and 3-acylation as may be desired; see e.g., US-A-3,562,257; 4,567,175 and 4,885,375 as well as European Patent Application EP.... (our ref.: A 635 EP)

entitled, "BENZOTHIAZEPINES," and filed on even date herewith.

In addition, the Benzothiazepine-potentiators having the vinyl chloride at positions 2 & 3 of the benzothiazepine nucleus can be made by processes known in the art; see e.g., US-A-3,895;006, 3,983,106 and 3,075,967.

The term "anti-cancer drug" reffers herein to a compound or composition which is cytotoxic particularly to include towards cancer cells or cancerous tissue. The anti-cancer drug may be selected from such types of compounds as anthracyclines, vinca alkaloids, or cis-Platinum compounds to include coordination compounds thereof. Desirably, the anti-cancer drug is an anthracycline, e.g., daunorubicin, or a vinca alkaloid, e.g., vincristine or vinblastin, especially an anthracycline.

In the practice of this invention, method embodiments are carried out such that the effect of the anti-cancer drug is potentiated. Accordingly, as is understood in the art, a suitable dosage or dosage regimen is employed to obtain the desired effect of the components employed in relation to the subject. Amounts of the Benzothiazepine-potentiator can thus vary in relation to amounts of the anti-cancer drug employed, but total drug dosages can be smaller than corresponding dosages of the anti-cancer drug alone. Therefore, with respect to composition embodiments of this invention, unit dosage formulations can be made accordingly smaller, oftimes substantially and surprisingly so, than corresponding unit dosage formulations of the anti-cancer drug if it were generally formulated alone.

In making the composition off this invention, methods known in the art can be employed. These include well-known powder-making methods, bead-making methods, tablet-making methods, capsule-making methods, syrup-making methods, and dragee-making methods, to include methods for delayed release of one or more of the medicaments of this invention as may be appropriate, methods for the making of an intravenously injectable solution or colloidal mixture, methods for the making of a sustainably releasing implantable article or device, methods for making an ointment such as a creme or a gel, and methods for the making of a composition suitable for transdermal application of components with the aid of a suitable carrier. Accordingly, composition embodiments of this invention can have suitable adjuvant(s) therewith to help achieve the desired end dosage form provided.

The following further illustrates this invention. Therein, parts and percentages are by weight unless otherwise specified.

## Example with Comparative

Samples of both sensitive and resistant K562/III cells were prepared by the method of Ahmed & Vasanthakumar, supra. The resistant cells were used as one form of control. The cells were suspended in a mixture of nine parts RPMI 1640 media (Hazelton Biologics, Inc.) and one part fetal bovine serum (FBS) (Hazelton Biologics, Inc.) at a concentration of 500,000 cells per mL.

To determine chemotherapeutic activity, individual samples were prepared and evaluated as follows:

To a mixture of 3.8 mL of the 1640 media and 0.38 mL of the FBS was added 0.1 mL of a mixture of daunorubicin at various concentrations in sterile water and 0.1 mL of a mixture of potentiator candidate at a concentration of 0.5 mM in the 1640 media. To the foregoing mixture was then added 1 mL of the cell suspension. A control having only the 0.1 mL of the daunorubicin mixture with 0.1 mL of the sterile water was similarly prepared. The resultant sample or control volumes were each 5 mL total.

The samples and controls were incubated for 48 hours at 37 degrees C. in the dark under an atmosphere of 5 percent carbon dioxide and 95 percent oxygen. At the end of this incubation, the samples and controls were counted by the Coulter counter method to determine cell densities. These cell densities provided the IC50 values reported. Mean IC50 and relative (rel.) IC50 values were calculated. The mean IC50 values were calculated by standard methods. Rel. IC50 values were calculated by dividing the IC50 values obtained for a particular treatment by the IC50 value of daunorubicin to obtain a quotient, and multiplying the quotient by the average of all IC50 values for all daunorubicin samples. This takes into account the various activities of different daunorubicin lots. Both mean and rel. IC50 values for daunorubicin for 11 trials were 1465 ± 299.

Anticonvulsant activity (AcA) was determined generally by the method of Zobrist et al., European Patent Application EP.... (our ref.: A 634 EP) entitled, "BENZOTHZAZEPINE ANTI-SEIZURE METHOD," and filed on even date herewith; see also US-A-4,879,289.

However, the AcA is reported on a scale from 0 to 5, with 0 being no observed activity and 5 being the most active.

Cardovascular activity (CvA) was determined by the use of isolated tissue. The CvA is reported on a scale from 0 to 5, with 0 being no observed activity and 5 being the most active.

Anti-depressant activity (AdA) was determined by visual observation. The AdA is reported on a scale

from 0 to 5, with 0 being no observed activity and 5 being the most active.

Table I identifies the Benzothiazepine-potentiator (B-P) compounds employed in this example, with substituent references being made to the formula I. The B-P compounds were employed as the listed salt. Table II lists results. The parenthetical number (#) is the number of trials carried out and used in determining the mean and rel. IC50 values. See also, **Figs. 1-20**.

TABLE I

| B-P compound | Q | R | Y: R' of OR', &c | R" | Salt |
|---|---|---|---|---|---|
| l-cis-DTZ | H | OMe | acetyl | R"1a | HCl |
| d-cis-ML1013 | H | OMe | valeryl | R"1a | fumerate |
| d-cis-ML1014 | H | OMe | isovaleryl | R"1a | fumerate |
| d-cis-ML1015 | H | OMe | pivalyl | R"1a | fumerate |
| d-cis-ML1016 | H | OMe | acetyl | R"1b | HCl |
| dl-cis-ML1017 | H | OMe | H | R"5 | HCl |
| dl-cis-ML1018 | H | OMe | acetyl | R"5 | HCl |
| dl-cis-ML1020 | H | OMe | H | R"3 | HCl |
| dl-cis-ML1021 | H | OMe | acetyl | R"3 | HCl |
| dl-cis-ML1047 | 8-Cl | OMe | pivalyl | R"1a | fumerate |
| dl-cis-ML1048 | H | OMe | H | R"6 | HCl |
| dl-cis-ML1063 | H | OMe | H | R"7 | HCl |
| dl-cis-ML1064 | H | OMe | acetyl | R"7 | HCl |
| dl-cis-ML1065 | H | OMe | H | +R"9a-X | Br/Cl |

TABLE I (Continued)

| B-P compound | Q | R | Y: R′ of OR′, &c | R" | Salt |
|---|---|---|---|---|---|
| dl-cis-ML1066 | H | OMe | acetyl | +R"9a-X | Br/Cl |
| dl-cis-ML1077 | H | Cl | H | R"1a | HCl |
| dl-cis-ML1078 | H | CF3 | H | R"1a | HCl |
| dl-cis-ML1079 | H | CH3 | H | R"1a | HCl |
| dl-cis-ML1080 | H | OMe | adamantylcarboxy | R"1a | fumerate |
| dl-cis-ML1082 | H | CF3 | H | R"5 | HCl |
| dl-trans-ML1096 | 8-Cl | OMe | H | R"5 | HCl |
| ML1097 | H | OMe | *Cl | R"1a | HCl |
| dl-cis-ML1098 | H | H | H | R"5 | HCl |
| dl-trans-ML1103 | H | OMe | H | R"5 | HCl |
| dl-cis-ML1104 | H | CN | H | R"5 | HCl |
| d-cis-TA3090 | 8-Cl | OMe | acetyl | R"1a | maleate |
| l-cis-TA3090 | 8-Cl | OMe | acetyl | R"1a | maleate |

*vinyl chloride at positions 2 & 3

TABLE II

| Potentiator | AcA | CvA | AdA | Mean IC50 | (#) | Rel. IC50 | (#) |
|---|---|---|---|---|---|---|---|
| *Verapamil | | | | 333±97 | (5) | 329±70 | (5) |
| *Diltiazem | 5 | 4 | 2 | 973±189 | (3) | 925±42 | (3) |
| l-cis-DTZ | 3 | | | 817±295 | (4) | 756±151 | (4) |
| d-cis-ML1013 | 0 | 1 | | 221±39 | (3) | 194±28 | (3) |
| d-cis-ML1014 | 1 | 1 | | 280±60 | (3) | 240±58 | (3) |
| d-cis-ML1015 | 0 | 1 | | 293±52 | (3) | 260±66 | (3) |
| d-cis-ML1016 | 5 | 2 | 1 | 333±37 | (3) | 288±58 | (3) |

TABLE II (Continued)

| Potentiator | AcA | CyA | AdA | Mean IC50 | (#) | Rel. IC50 | (#) |
|---|---|---|---|---|---|---|---|
| dl-cis-ML1017 | 5 | 1 | 1 | 1013±184 | (2) | 933±45 | (2) |
| dl-cis-ML1018 | 5 | 0 | 0 | 772±160 | (3) | 679±216 | (3) |
| dl-cis-ML1020 | 4 | 1 | 2 | 1305±111 | (2) | 1230±180 | (2) |
| dl-cis-ML1021 | 4 | 1 | 0 | 606±55 | (2) | 570±81 | (2) |
| dl-cis-ML1047 | 0 | 1 | | 1238 | (1) | 1462 | (1) |
| dl-cis-ML1048 | 5 | | | 849 | (1) | 1002 | (1) |
| dl-cis-ML1063 | 1 | | | 933 | (1) | 1140 | (1) |
| dl-cis-ML1064 | 1 | | | 1091±28 | (2) | 1357±9 | (2) |
| dl-cis-ML1065 | 0 | | | 1312±51 | (2) | 1635±93 | (2) |
| dl-cis-ML1066 | 0 | | | 1289±115 | (2) | 1607±172 | (2) |
| dl-cis-ML1077 | 5 | | | 1453±166 | (2) | 1744±309 | (2) |
| dl-cis-ML1078 | 5 | | | 445±58 | (2) | 535±102 | (2) |
| dl-cis-ML1079 | 3 | | | 1281±95 | (2) | 1534±210 | (2) |
| dl-cis-ML1080 | | | | 843±350 | (3) | 1035±466 | (3) |
| dl-cis-ML1082 | 5 | | | 416±20 | (2) | 464±37 | (2) |
| dl-trans-ML1096 | | | | 822±8 | (2) | 979±53 | (2) |
| ML1097 | | | | 1421±363 | (2) | 1545±203 | (2) |
| dl-cis-ML1098 | | | | ~~582±8~~ | (2) | 999±70 | (2) |
| dl-trans-ML1103 | | | | 299±12 | (2) | 352±42 | (2) |
| dl-cis-ML1104 | | | | 395±14 | (2) | 463±21 | (2) |
| d-cis-TA3090 | 2 | 5 | 1 | 1297±4 | (2) | 1053±30 | (2) |
| l-cis-TA3090 | 2 | | | 672±274 | (5) | 551±223 | (5) |

*Comparative

In further comparison with the foregiong, compounds having the following substituents as in the formula I provided the following effects with daunorubicin on the K562/III cell samples by the same methodology, the first compound being in the form of its hydrochloride salt, as listed in Table III.

TABLE III

| Q | R | R' | R'' | Mean/Rel. IC50s (#) |
|---|---|---|---|---|
| H | OMe | H | R''1a | 1827±307/2101±10 (2) |
| H | OMe | acetyl | 3-methylbut-2-enyl | 1923±174/1630±45 (3) |

## Claims

1. The use of a benzothiazepine for preparing a pharmaceutical composition for treating cancer by potentiating an anti-cancer drug effect in a subject.
2. The use of claim 1, wherein the Benzothiazepine-potentiator is at least one compound, or a pharmacuetically acceptable salt thereof, represented by the following general formula:

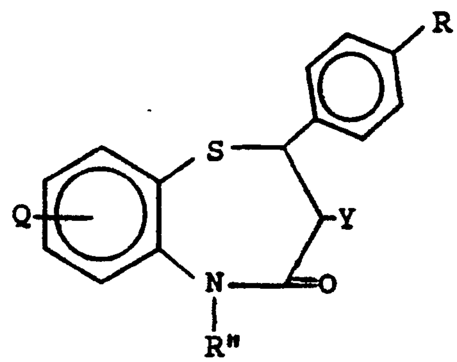

(I)

wherein
Q is H or halo;
R is H, lower alkoxy, lower haloalkyl, CN, lower alkyl or halo;
Y is
OR′, wherein R′ is H or alkylacyl, provided that then there is full saturation between carbons 2 & 3 of the benzothiazepine nucleus and 2,3-dihydro-functionality thereat as well, or
Cl, provided that then there is ethylenic unsaturation between positions 2 & 3 of the benzothiazepine nucleus, and
R″ is 2-[di(lower alkyl)amino]ethyl (R″1), 3-[di(lower alkyl)amino]propyl (R″2), 2-(pyrrolidino)ethyl (R″3), 3-(pyrrolidino)propyl (R″4), 2-(piperidino)ethyl (R″5), 3-(piperidino)propyl (R″6), 2-(morpholino)ethyl (R″7), 3-(morhpolino)propyl (R″8) or (N-pyridinium)alkyl with a suitable counterion being present (+R″9-X).
3. The use of claim 2, wherein said potentiator is at least one B-P, or a pharmaceutically acceptable salt thereof, selected from the group consisting of the following B-Ps, identified as follows with respect to compounds of the formula I:

| B-P compound | Q | R | Y: R′ of OR′ &C | R″ |
|---|---|---|---|---|
| 1-cis-DTZ | H | OMe | acetyl | R″1a, |
| cis-ML1013 | H | OMe | valeryl | R″1a, |
| cis-ML1014 | H | OMe | isovaleryl | R″1a, |
| cis-ML1015 | H | OMe | pivalyl | R″1a, |
| cis-ML1016 | H | OMe | acetyl | R″1b, |
| cis-ML1017 | H | OMe | H | R″5, |
| cis-ML1018 | H | OMe | acetyl | R″5, |
| cis-ML1020 | H | OMe | H | R″3, |
| cis-ML1021 | H | OMe | acetyl | R″3, |
| cis-ML1047 | 8-Cl | OMe | pivalyl | R″1a, |
| cis-ML1048 | H | OMe | H | R″6, |
| cis-ML1063 | H | OMe | H | R″7, |
| cis-ML1064 | H | OMe | acetyl | R″7, |
| cis-ML1065 | H | OMe | H | +R″9a-X, |
| cis-ML1066 | H | OMe | acetyl | +R″9a-X, |
| cis-ML1077 | H | Cl | H | R″1a, |
| cis-ML1078 | H | CF3 | H | R″1a, |
| cis-ML1079 | H | CH3 | H | R″1a, |
| cis-ML1080 | H | OMe | adamantylcarboxy | R″1a, |
| cis-ML1082 | H | CF3 | H | R″5, |
| trans-ML1096 | 8-Cl | OMe | H | R″5, |
| ML1097 | H | OMe | *Cl | R″1a, |
| cis-ML1098 | H | H | H | R″5, |
| trans-ML1103 | H | OMe | H | R″5, |
| cis-ML1104HCNHR″5 & | | | | |
| cis-TA3090 | 8-Cl | OMe | acetyl | R″1a, |

(* vinyl chloride at positions 2,3)

further wherein
OMe is methoxy;
CF3 is trifluoromethyl;
CH3 is methyl;
R″1a is 2-(dimethylamino)ethyl,
R″1b is 2-(diisopropylamino)ethyl, and
+R″9a-X is 2-(N-pyridinium)ethyl with a bromide and/or chloride couterion being present.

4. The use of claim 3, wherein said potentiator is l-cis-DTZ, or a pharmaceutically acceptable salt thereof.

5. The use of claim 3, wherein said potentiator is cis-TA3090, or a pharmaceutically acceptable salt thereof.

6. The use of claim 5, wherein said potentiator is d-cis-TA3090, or a pharmaceutically acceptable salt thereof.

7. The use of claim 5, wherein said potentiator is l-cis-TA3090, or a pharmaceutically acceptable salt thereof.

8. The use of claim 3, wherein said potentiator is at least one B-P, or a pharmaceutically acceptable salt thereof, selected from the group consisting of d-cis-ML1013, d-cis-ML1014, d-cis-ML1015, d-cis-ML1016, cis-ML1021, cis-ML1078, d-cis-ML1082, d-trans-ML1103 and d-cis-ML1104.

9. The use of a potentiator being selected from at least one of the group consisting of:
l-cis-8-chloro-5-[2-(dimethylamino)ethyl]-2,3-dihydro3-hydroxy-2-(4-methoxyphenyl)-1,5-benzothiazepin-4-(5H)-one acetate (ester);
d-cis-5-[2-(dimethylamino)ethyl]-2,3-dihydro-3-hydroxy-2-(4-methoxyphenyl)-1,5-benzothiazepin-4(5H)-one valerylate (ester);
d-cis-5-[2-(dimethylamino)ethyl]-2,3-dihydro-3-hydroxy-2-(4-methoxyphenyl)-1,5-benzothiazepin-4(5H)-one isovalerylate (ester);
d-cis-5-[2-(dimethylamino)ethyl]-2,3-dihydro-3-hydroxy-2-(4-methoxyphenyl)-1,5-benzothiazepin-4(5H)-one

pivalate (ester);
d-cis-5-[2-(diisopropylamino)ethyl]-2,3-dihydro-3-hydroxy-2-(4-methoxyphenyl)- 1,5-benzothiazepin-4(5H)-one acetate (ester);
dl-cis-5-{2-[(pyrrolidino)ethyl]amino}-2,3-dihydro-3-hydroxy-2-(4-methoxyphenyl)-1,5-benzothiazepin-4(5H)-one acetate (ester);
dl-cis-5-[2-(dimethylamino)ethyl]-2,3-dihydro-3-hydroxy-2-(4-trifluoromethylphenyl)-1,5-benzothiazepin-4(5H)-one;
d-cis-5-[2-(piperidino)ethyl]-2,3-dihydro-3-hydroxy-2-(4-trifluorophenyl)-1,5-benzothiazepin-4(5H)-one;
d-trans-5-[2-(piperidino)ethyl]-2,3-dihydro-3-hydroxy-2-(4-methoxyphenyl)-1,5-benzothiazepin-4(5H)-one, and
d-cis-5-[2-(piperidino)ethyl]-2,3-dihydro-3-hydroxy-2-(4-cyanophenyl)-1,5-benzothiazepin-4(5R)-one, and pharmaceutically acceptable salt(s) thereof for preparing a pharmaceutical composition for treating cancer by potentiating the anti-cancer effect of daunorubicin in a human patent.

10. A pharmaceutical composition useful for treatment of a cancer comprising a Benzothiazepine-potentiator in combination with an anti-cancer drug.

11. The composition of claim 10, wherein the Bensothiazepine-potentiator is at least one compound, or a pharmacuetically acceptable salt thereof, represented by the following general formula:

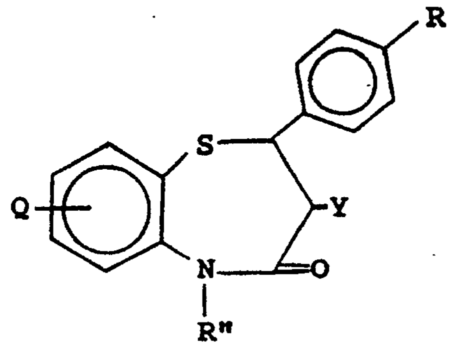

(I)

wherein
Q is H or halo;
R is H, lower alkoxy, lower haloalkyl, CN, lower alkyl or halo;
Y is
OR′, wherein R′ is H or alkylacyl, provided that then there is full saturation between carbons 2 & 3 of the benzothiazepine nucleus and 2,3-dihydro-functionality thereat as well, or
Cl, provided that then there is ethylenic unsaturation between positions 2 & 3 of the benzothiazepine nucleus, and
R″ is 2-[di(lower alkyl)amino]ethyl (R″1), 3-[di(lower alkyl)amino]propyl (R″2), 2-(pyrrolidino)ethyl (R″3), 3-(pyrrolidino)propyl (R″4), 2-(piperidino)ethyl (R″5), 3-(piperidino)propyl (R″6), 2-(morpholino)ethyl (R″7), 3-(morhpolino)propyl (R″8) or (N-pyridinium)alkyl with a suitable counterion being present (+R″9-X).

Fig. 1

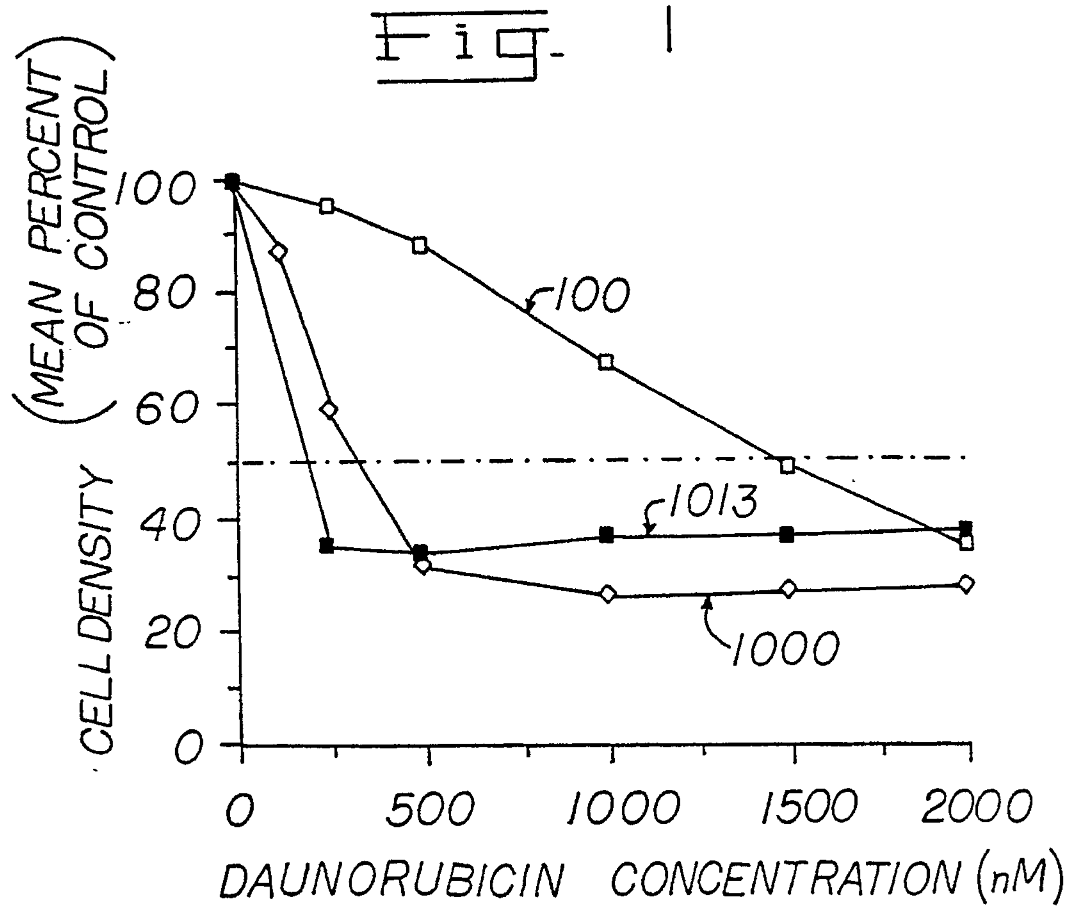
CELL DENSITY (MEAN PERCENT OF CONTROL)
100
80
60
40
20
0
100
1013
1000
0
500
1000
1500
2000
DAUNORUBICIN CONCENTRATION (nM)

Fig. 2

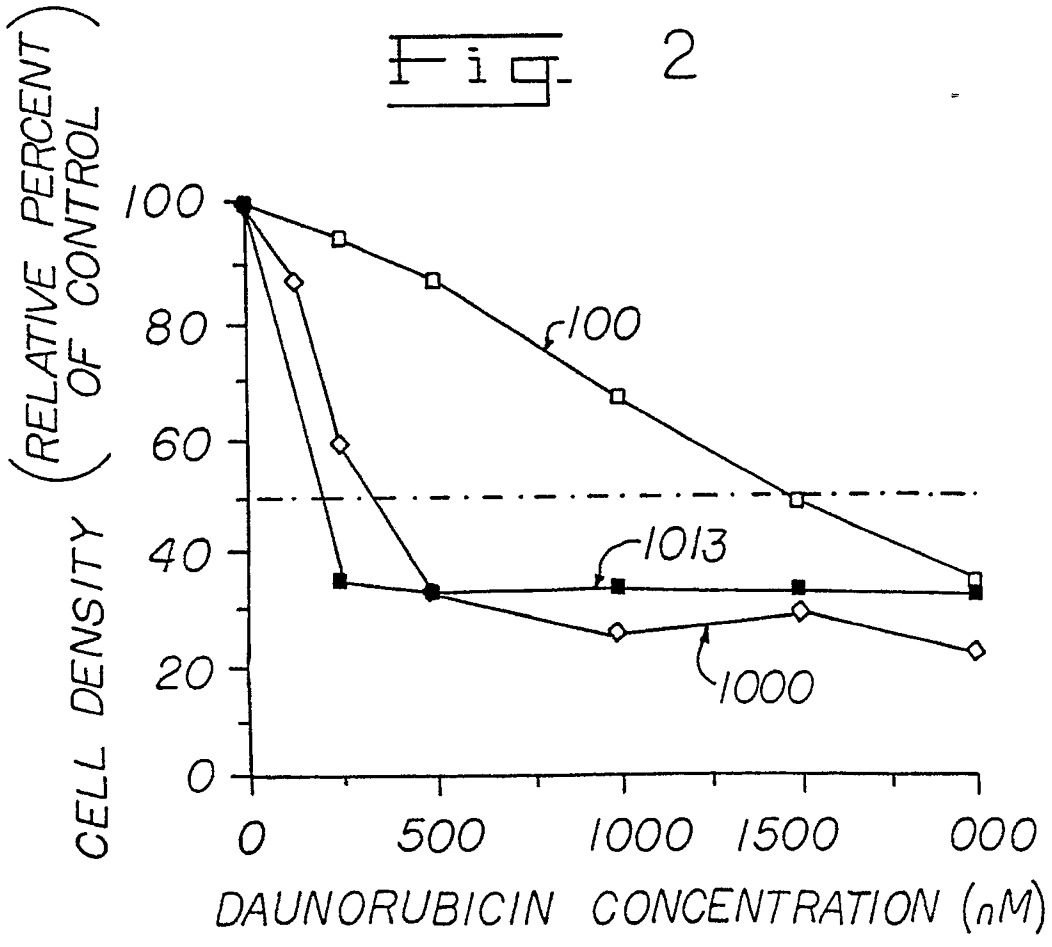
CELL DENSITY (RELATIVE PERCENT OF CONTROL)
100
80
60
40
20
0
100
1013
1000
0
500
1000
1500
000
DAUNORUBICIN CONCENTRATION (nM)

Fig. 3

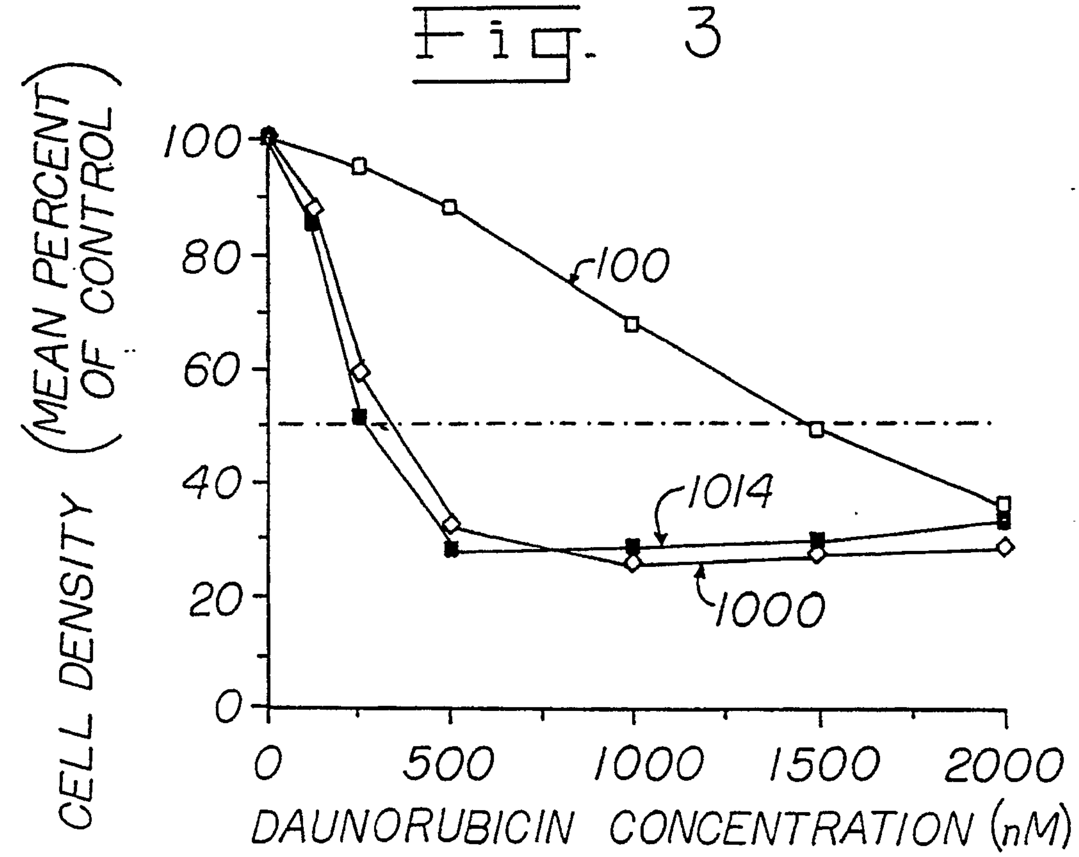
CELL DENSITY (MEAN PERCENT OF CONTROL)
100
80
60
40
20
0
100
1014
1000
0
500
1000
1500
2000
DAUNORUBICIN CONCENTRATION (nM)

Fig. 4

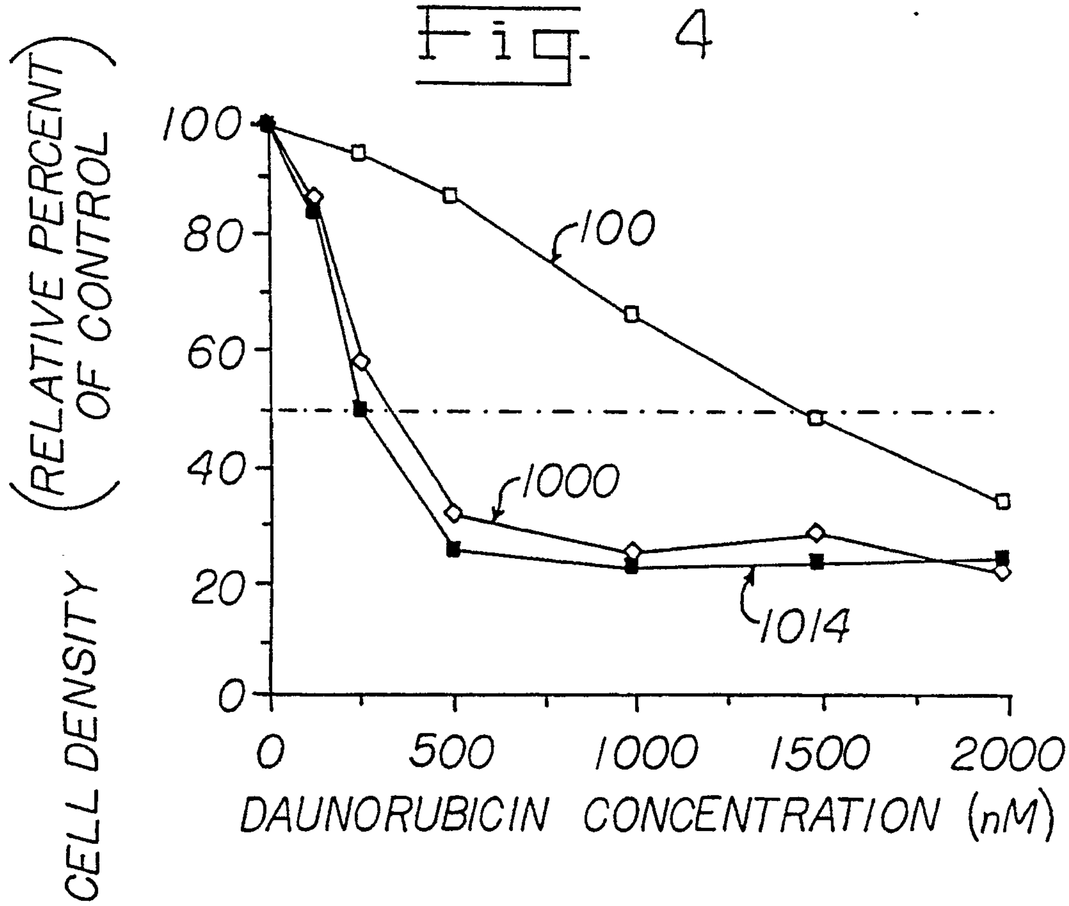
CELL DENSITY (RELATIVE PERCENT OF CONTROL)
100
80
60
40
20
0
100
1000
1014
0
500
1000
1500
2000
DAUNORUBICIN CONCENTRATION (nM)

Fig. 5

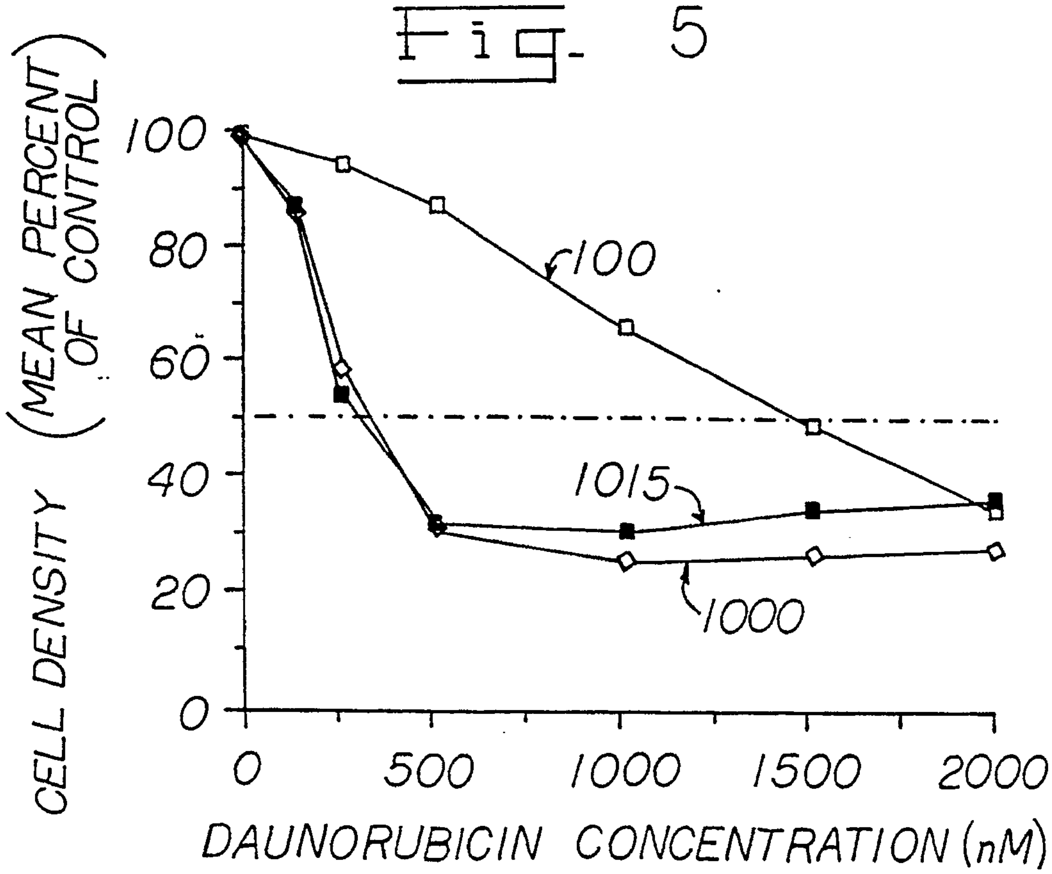
CELL DENSITY (MEAN PERCENT OF CONTROL)
100
80
60
40
20
0
100
1015
1000
0
500
1000
1500
2000
DAUNORUBICIN CONCENTRATION (nM)

Fig. 6

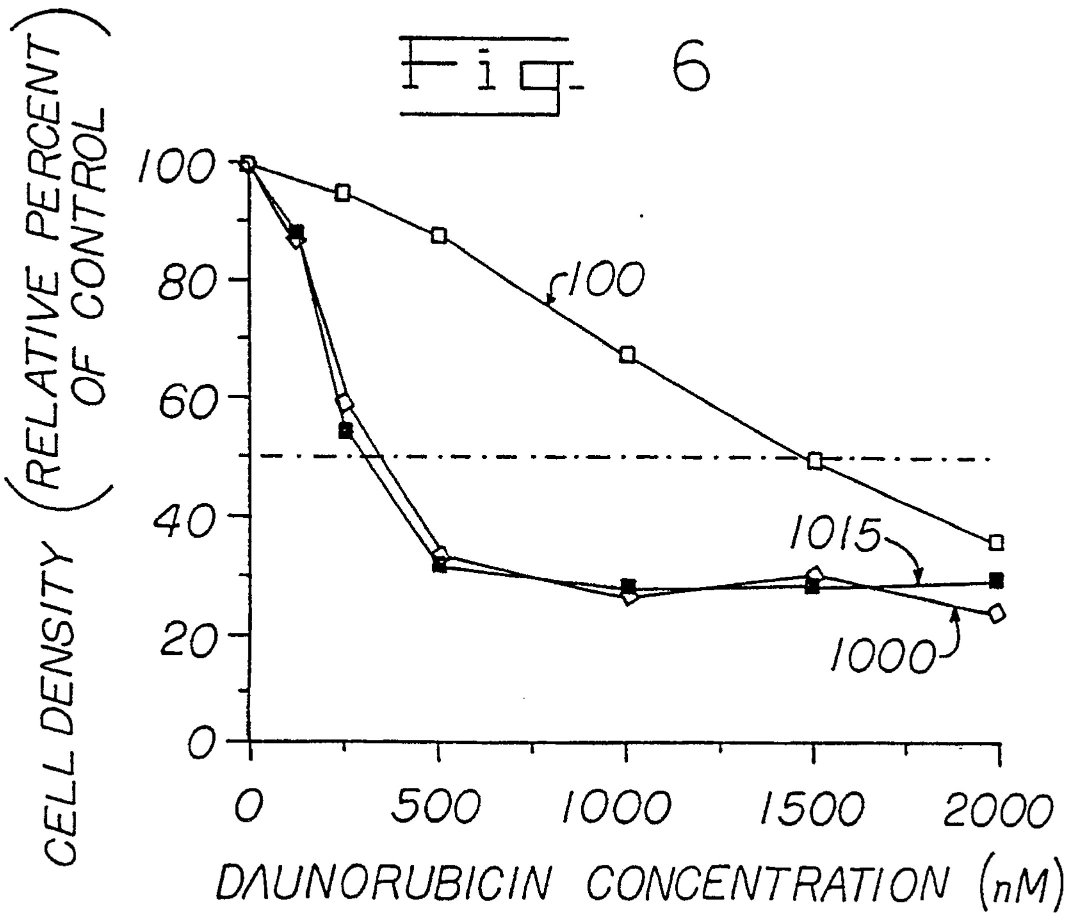
CELL DENSITY (RELATIVE PERCENT OF CONTROL)
100
80
60
40
20
0
100
1015
1000
0
500
1000
1500
2000
DAUNORUBICIN CONCENTRATION (nM)

Fig. 7

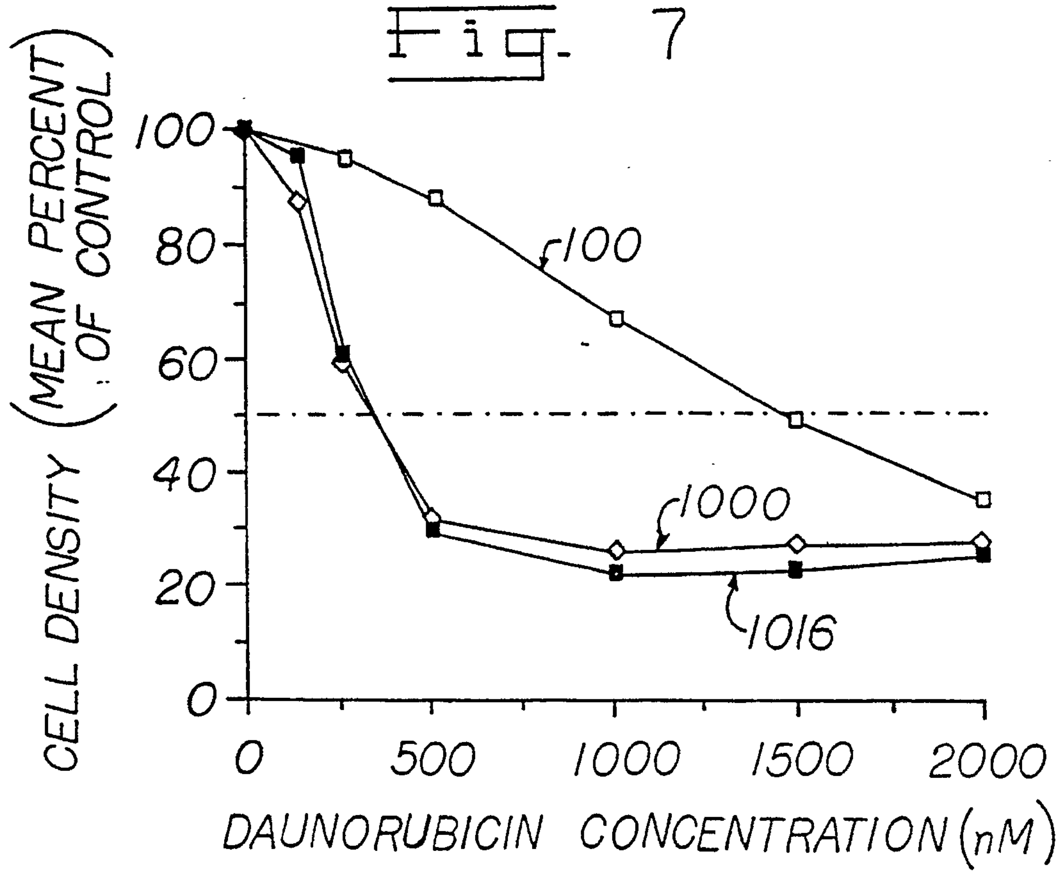
CELL DENSITY (MEAN PERCENT OF CONTROL)
100
80
60
40
20
0
100
1000
1016
0
500
1000
1500
2000
DAUNORUBICIN CONCENTRATION (nM)

Fig. 8

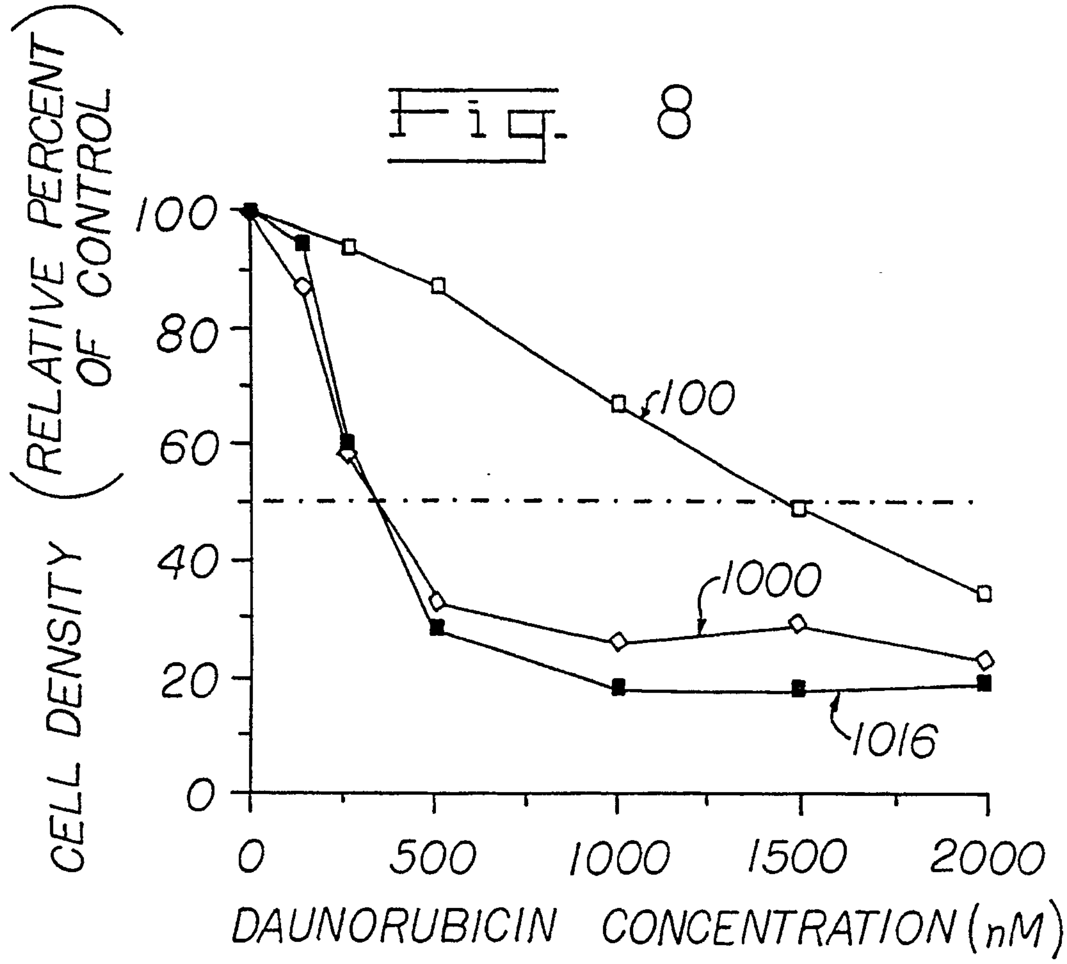
CELL DENSITY (RELATIVE PERCENT OF CONTROL)
100
80
60
40
20
0
100
1000
1016
0
500
1000
1500
2000
DAUNORUBICIN CONCENTRATION (nM)

Fig. 9

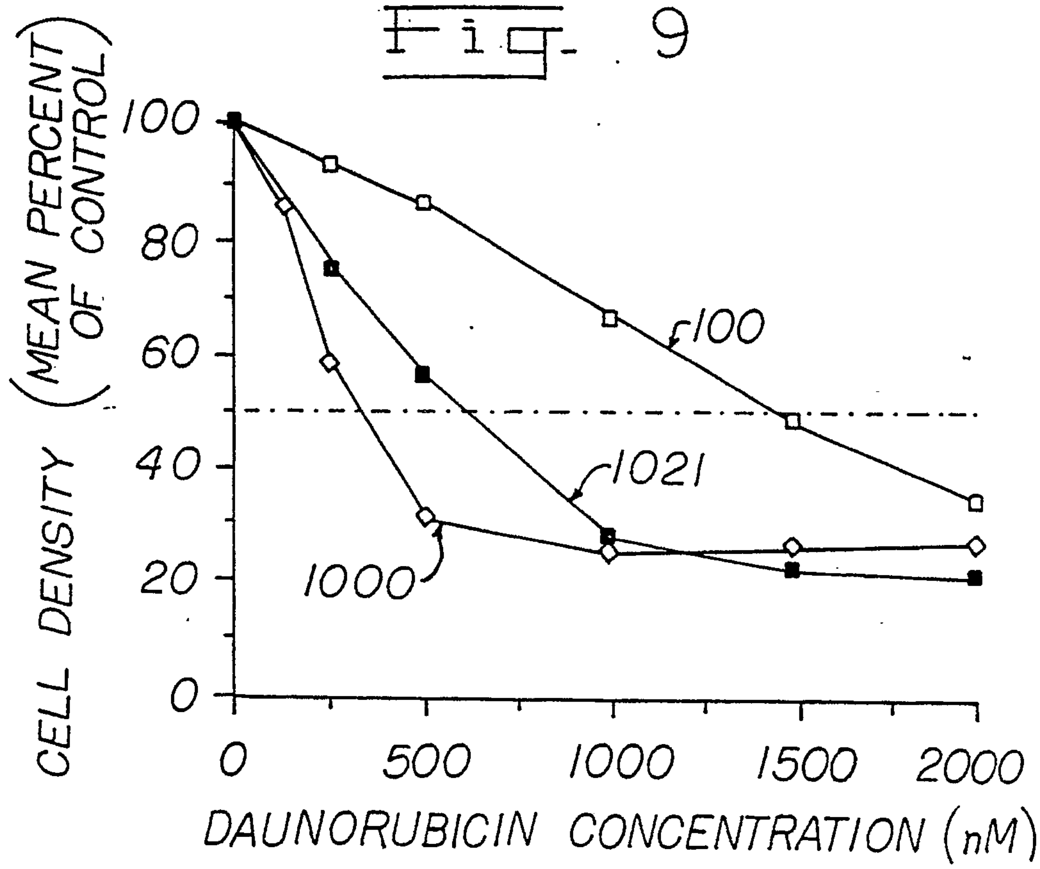
100
80
60
40
20
0
CELL DENSITY (MEAN PERCENT OF CONTROL)
100
1021
1000
0
500
1000
1500
2000
DAUNORUBICIN CONCENTRATION (nM)

Fig. 10

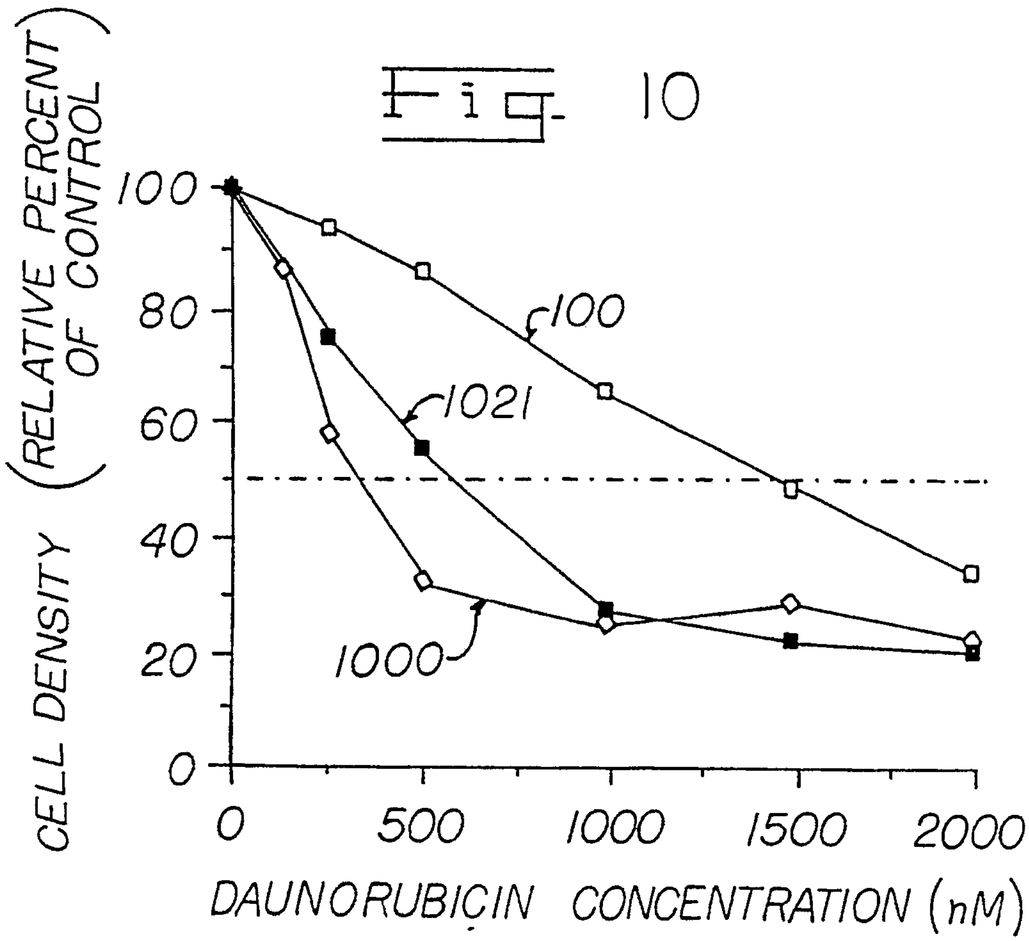
100
80
60
40
20
0
CELL DENSITY (RELATIVE PERCENT OF CONTROL)
100
1021
1000
0
500
1000
1500
2000
DAUNORUBICIN CONCENTRATION (nM)

Fig. 11

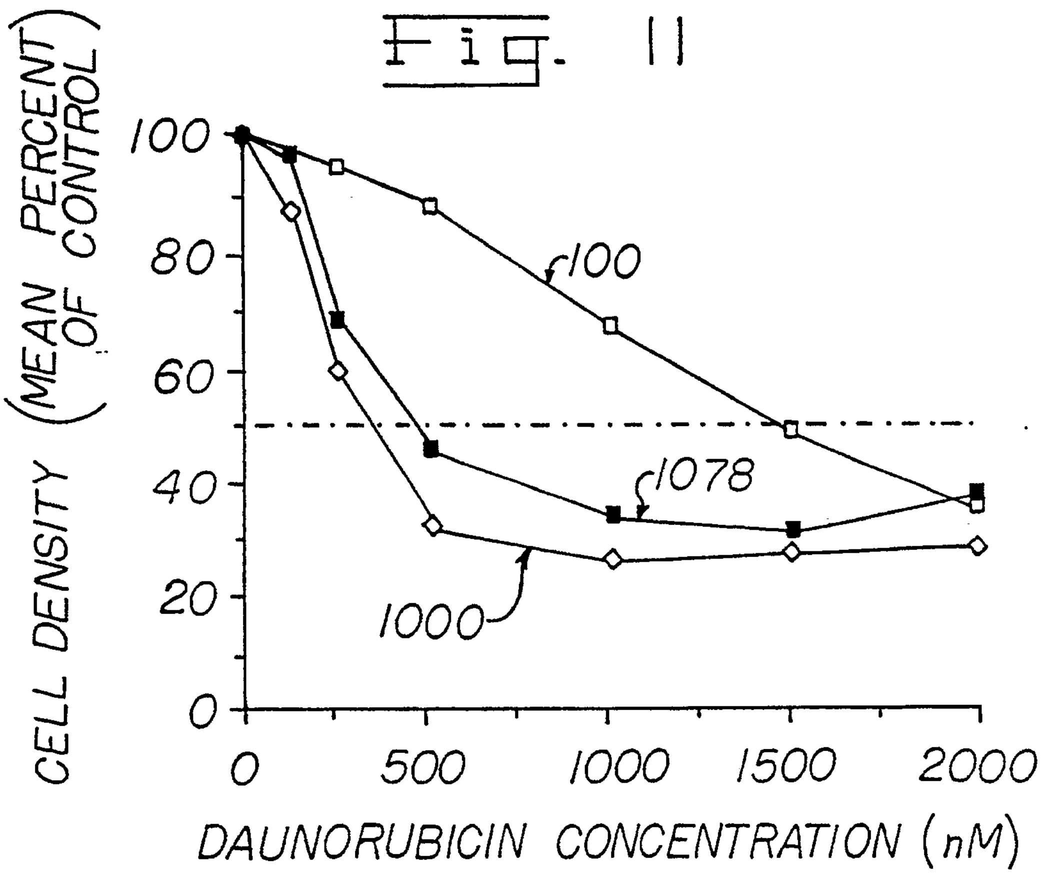
CELL DENSITY (MEAN PERCENT OF CONTROL)
100
80
60
40
20
0
100
1078
1000
0
500
1000
1500
2000
DAUNORUBICIN CONCENTRATION (nM)

Fig. 12

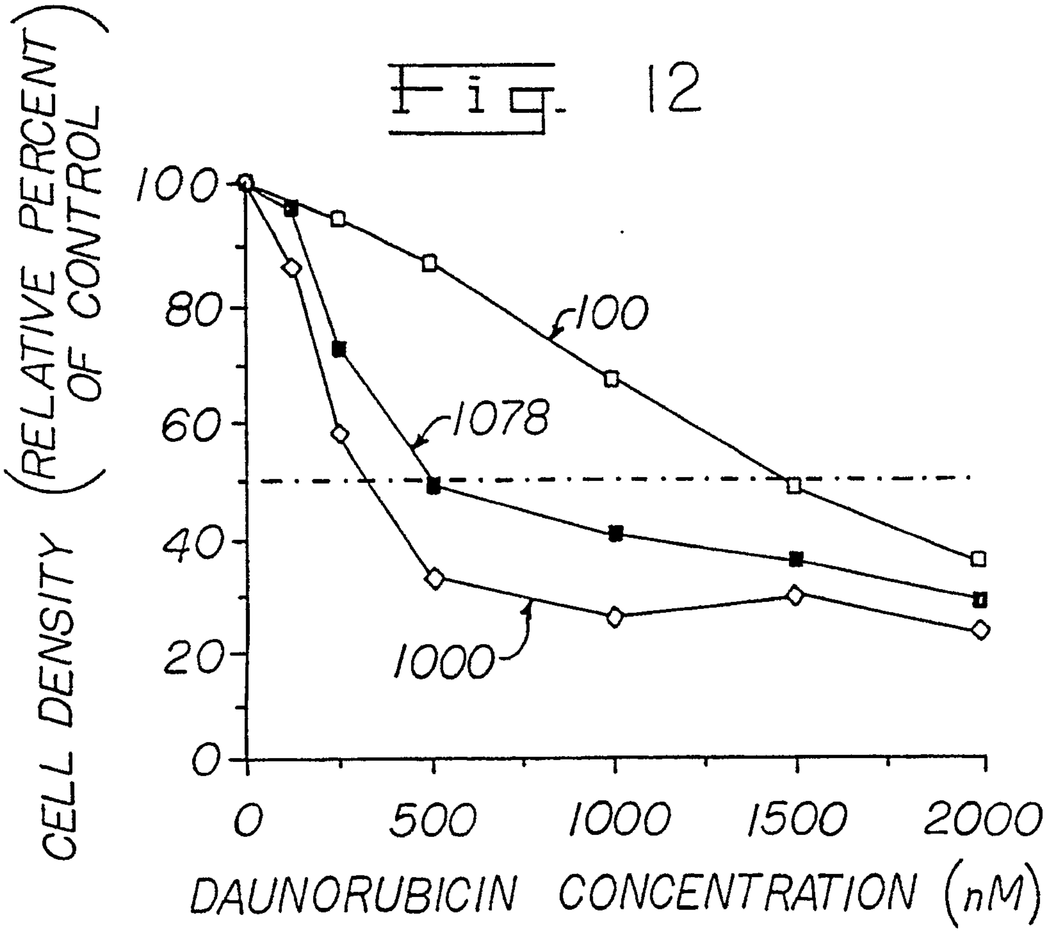
CELL DENSITY (RELATIVE PERCENT OF CONTROL)
100
80
60
40
20
0
100
1078
1000
0
500
1000
1500
2000
DAUNORUBICIN CONCENTRATION (nM)

Fig. 13

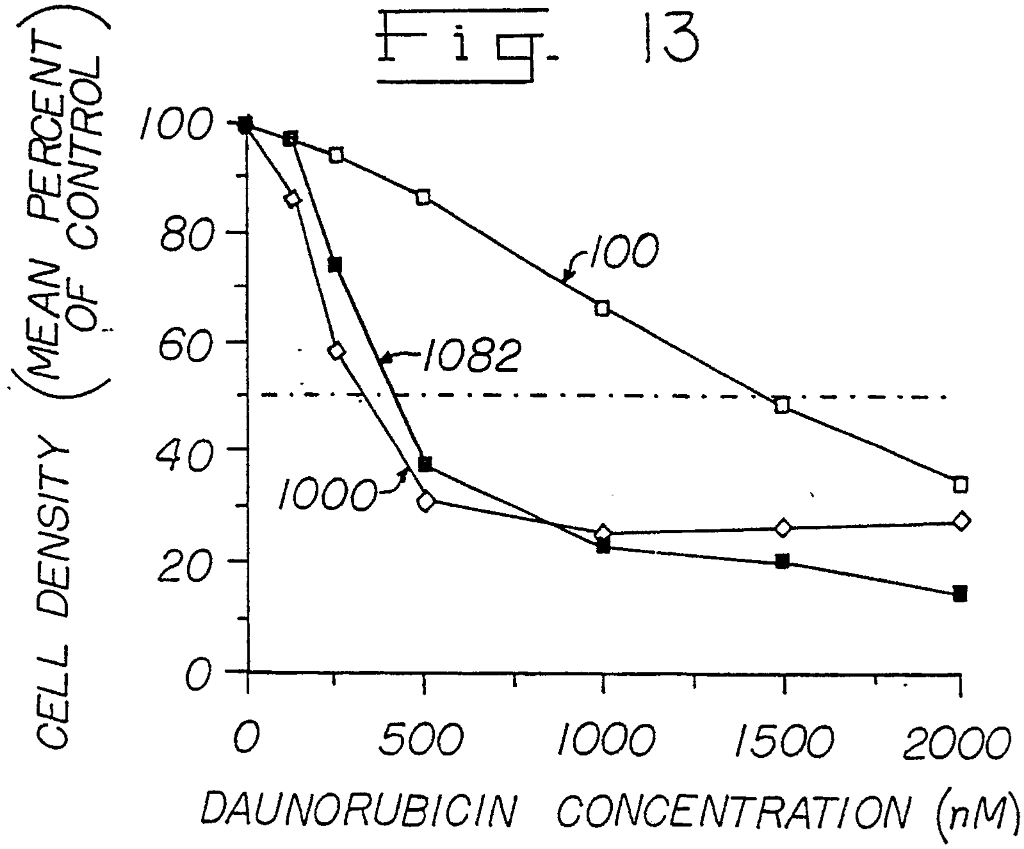
CELL DENSITY (MEAN PERCENT OF CONTROL)
100
80
60
40
20
0
100
1082
1000
0
500
1000
1500
2000
DAUNORUBICIN CONCENTRATION (nM)

Fig. 14

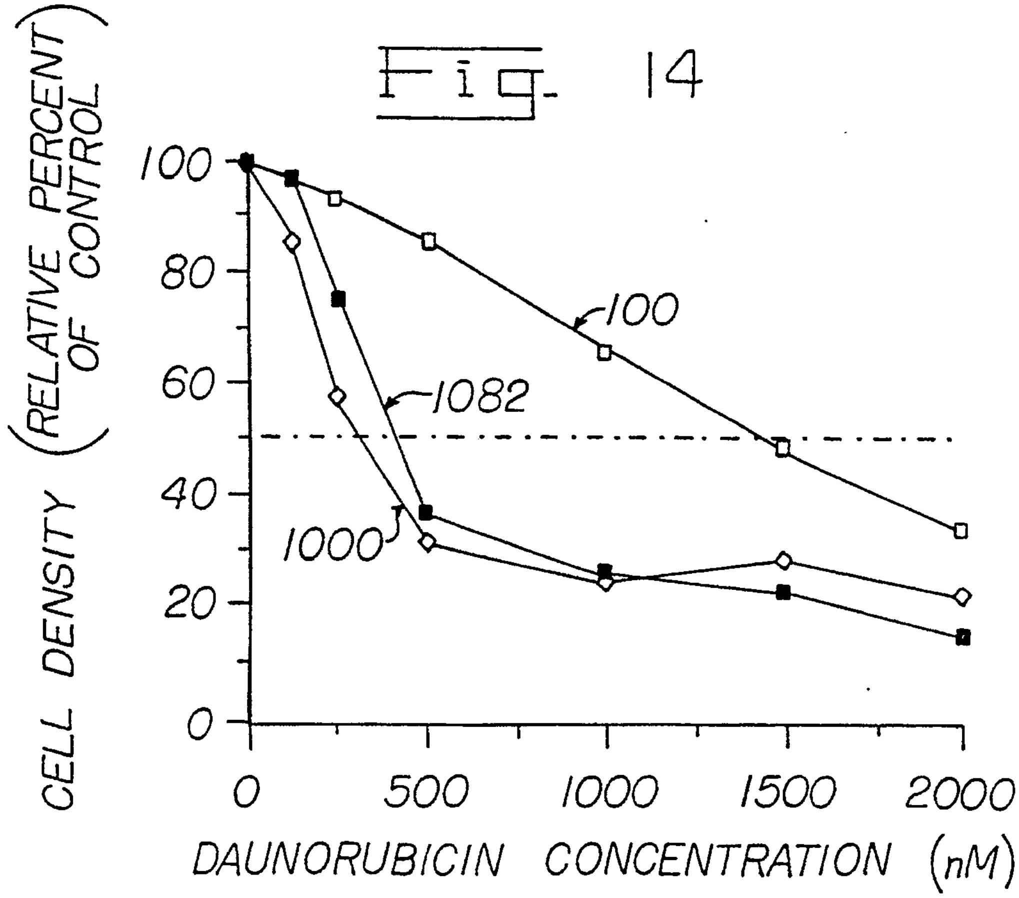
CELL DENSITY (RELATIVE PERCENT OF CONTROL)
100
80
60
40
20
0
100
1082
1000
0
500
1000
1500
2000
DAUNORUBICIN CONCENTRATION (nM)

Fig. 15

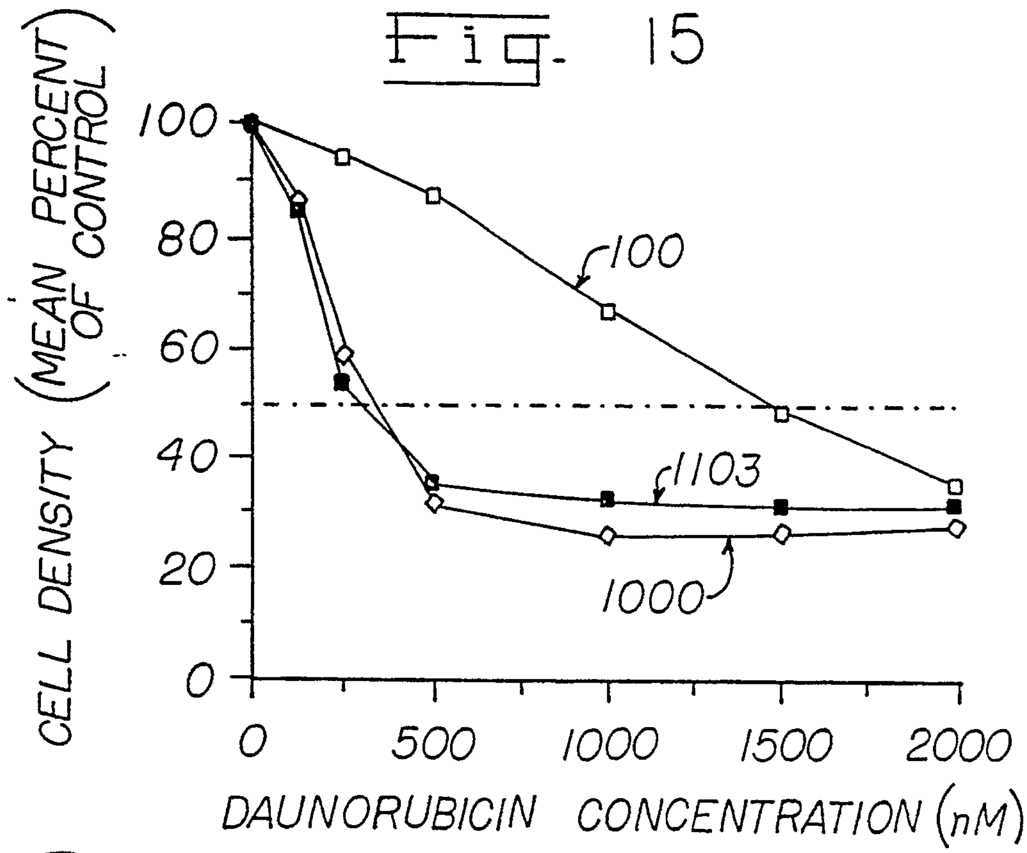
CELL DENSITY (MEAN PERCENT OF CONTROL)
100
80
60
40
20
0
100
1103
1000
0
500
1000
1500
2000
DAUNORUBICIN CONCENTRATION (nM)

Fig. 16

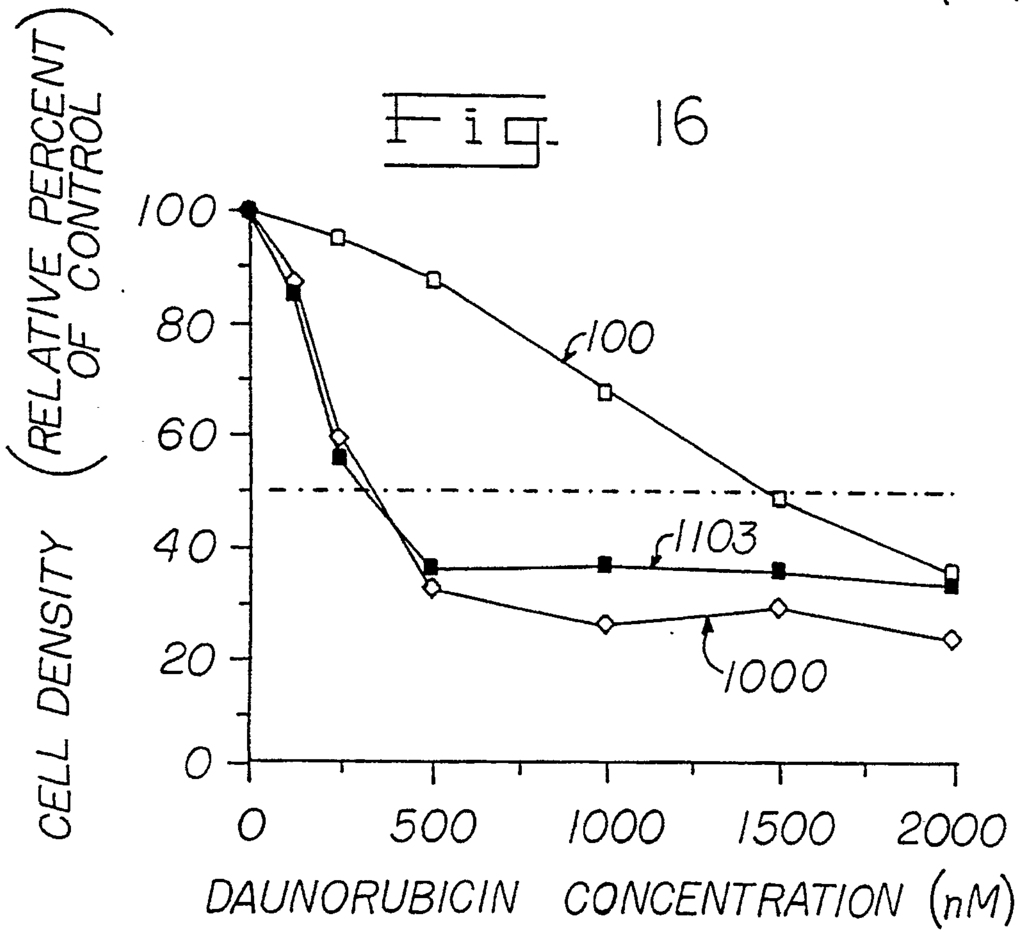
CELL DENSITY (RELATIVE PERCENT OF CONTROL)
100
80
60
40
20
0
100
1103
1000
0
500
1000
1500
2000
DAUNORUBICIN CONCENTRATION (nM)

Fig. 17

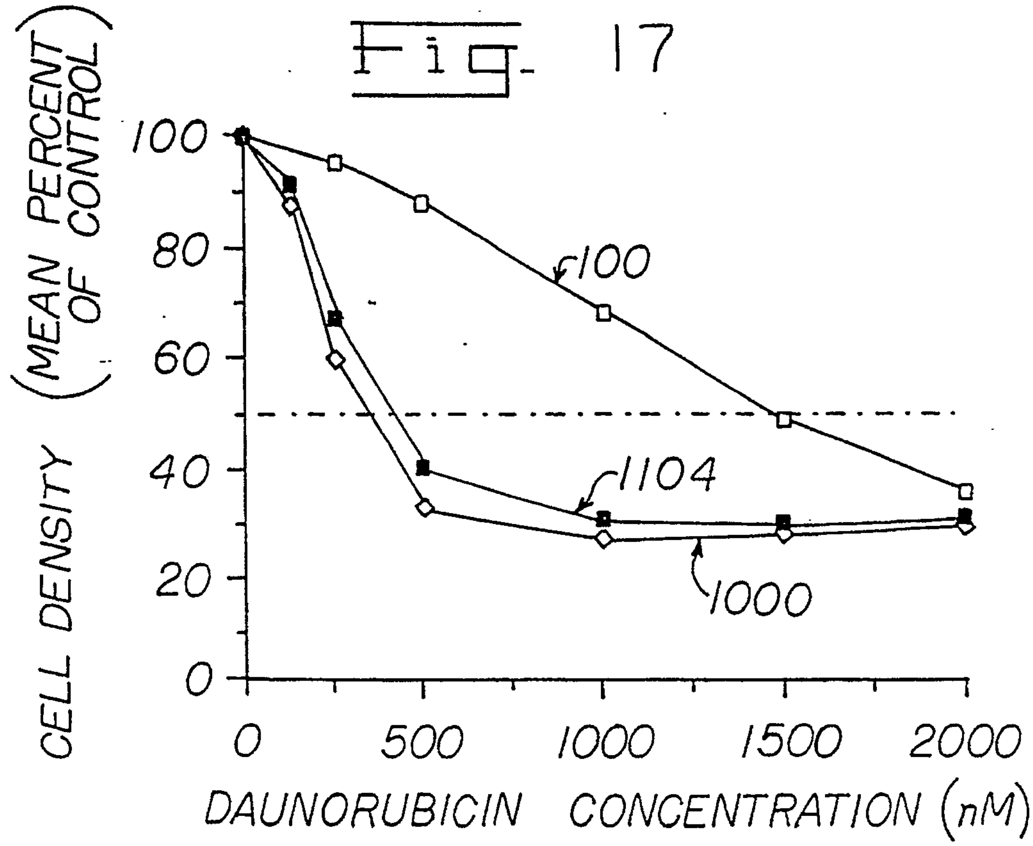
CELL DENSITY (MEAN PERCENT OF CONTROL)
100
80
60
40
20
0
100
1104
1000
0
500
1000
1500
2000
DAUNORUBICIN CONCENTRATION (nM)

Fig. 18

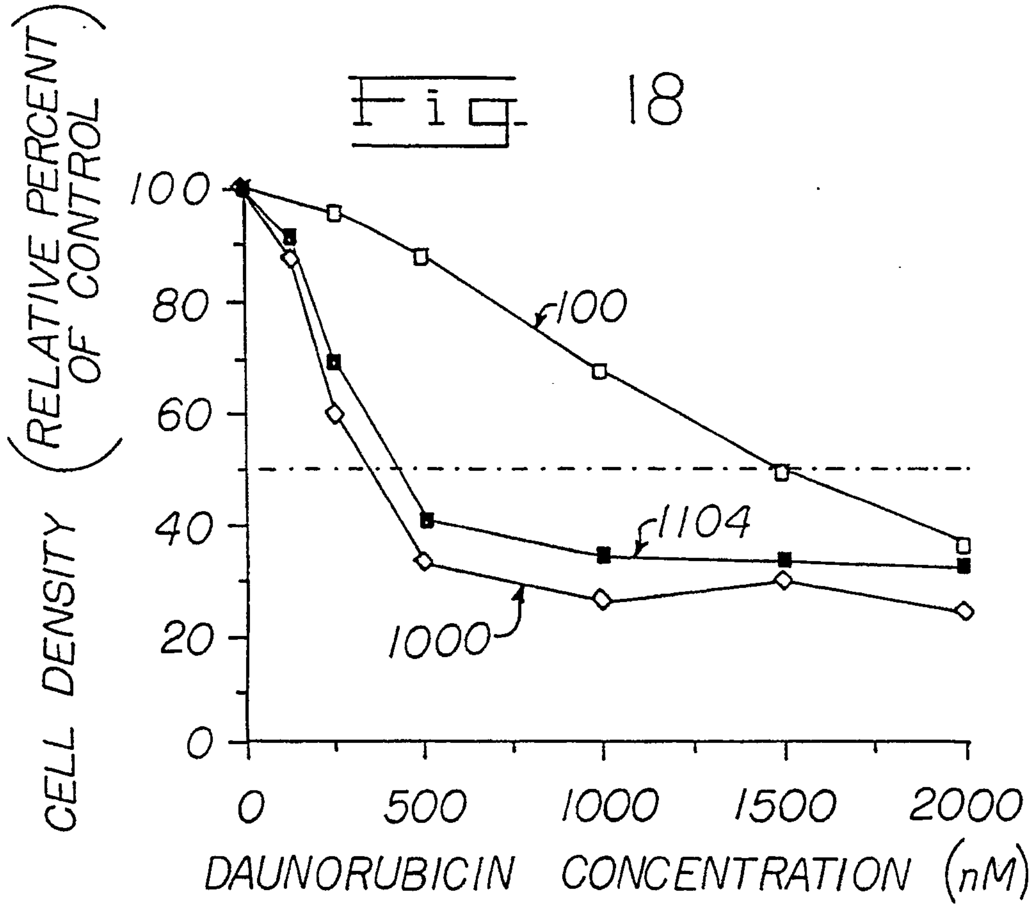
CELL DENSITY (RELATIVE PERCENT OF CONTROL)
100
80
60
40
20
0
100
1104
1000
0
500
1000
1500
2000
DAUNORUBICIN CONCENTRATION (nM)

Fig. 19

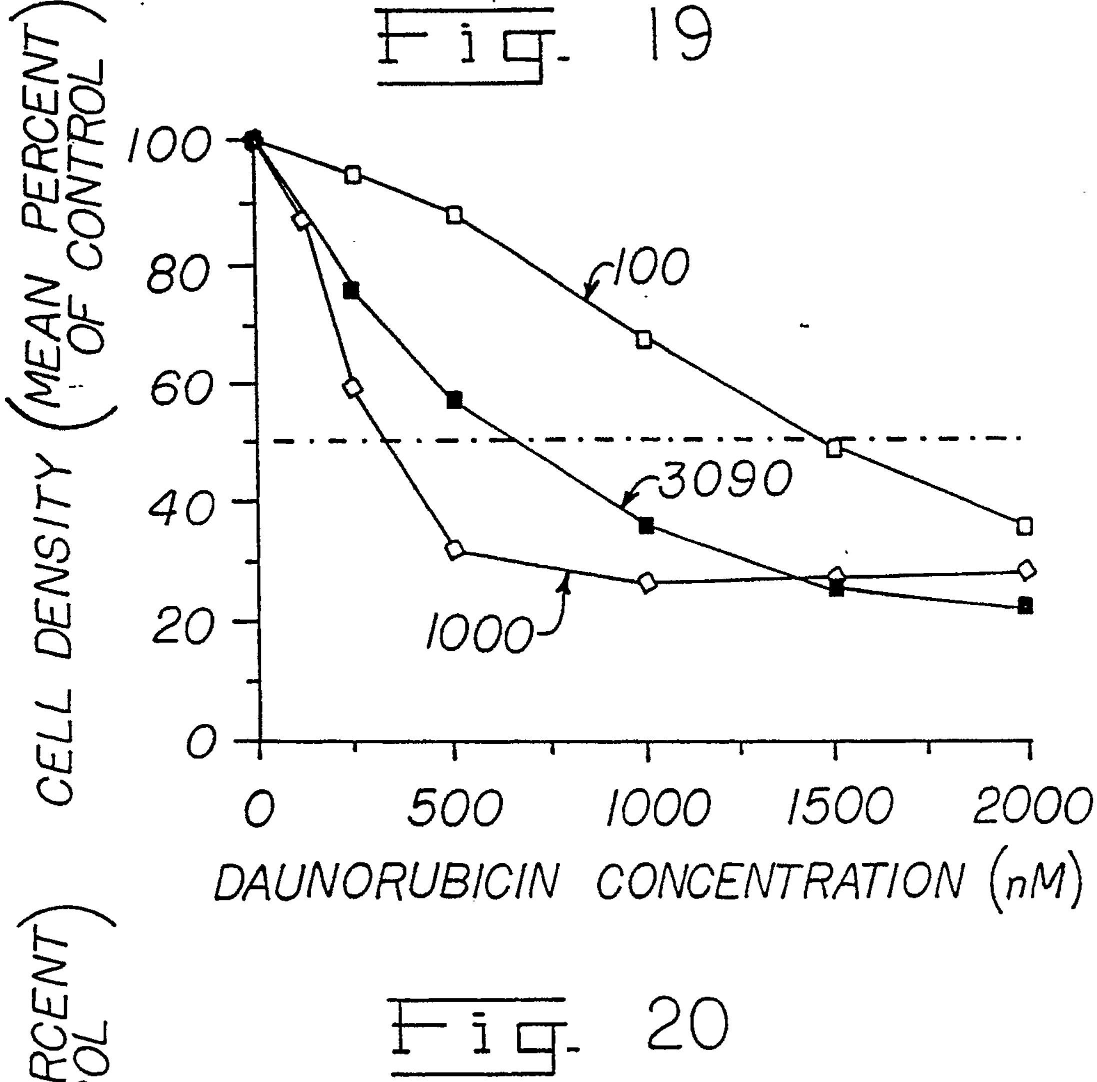
CELL DENSITY (MEAN PERCENT OF CONTROL)
100
80
60
40
20
0
100
3090
1000
0
500
1000
1500
2000
DAUNORUBICIN CONCENTRATION (nM)

Fig. 20

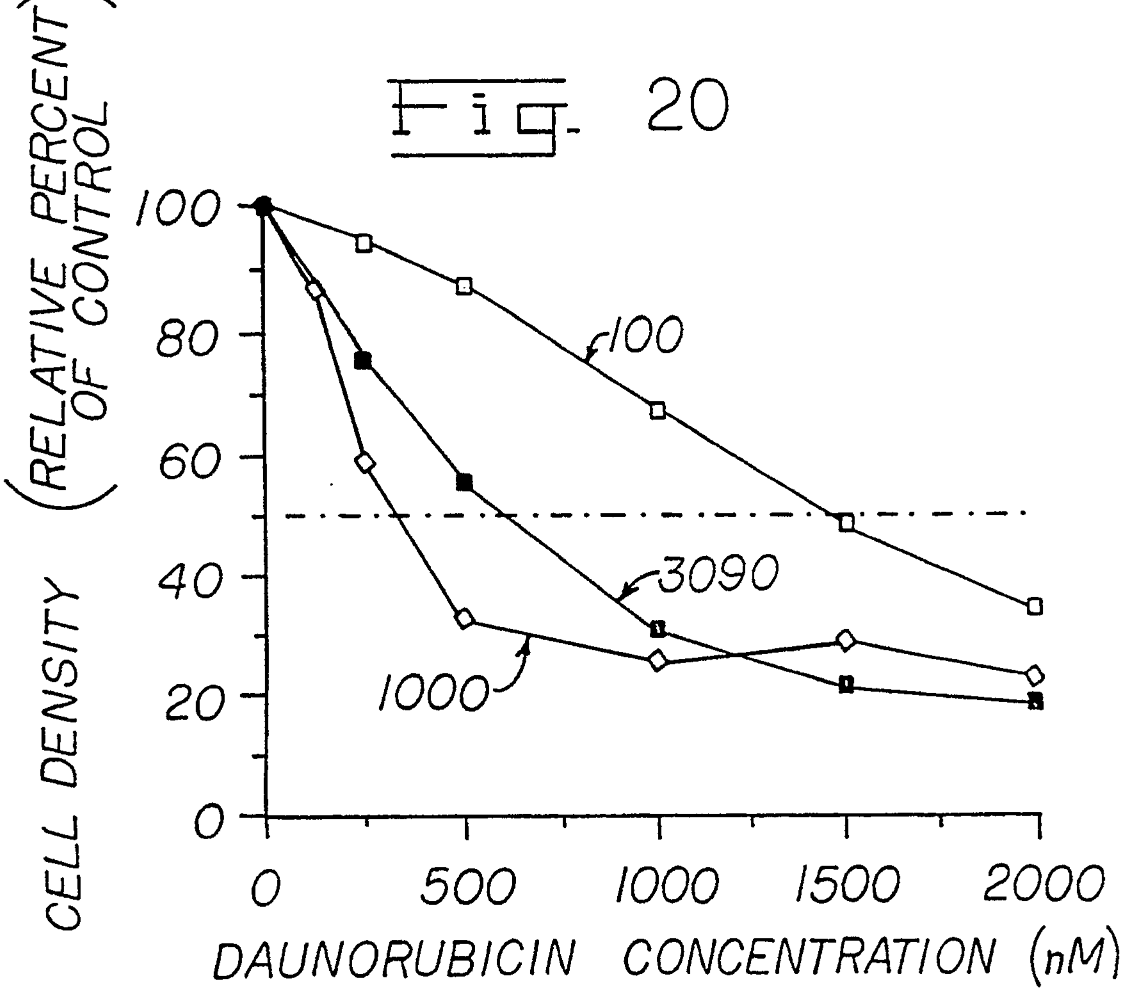
CELL DENSITY (RELATIVE PERCENT OF CONTROL)
100
80
60
40
20
0
100
3090
1000
0
500
1000
1500
2000
DAUNORUBICIN CONCENTRATION (nM)